**Europäisches Patentamt**

**European Patent Office**

**Office européen des brevets**

(19)

(11) Veröffentlichungsnummer: **0 003 340**
A2

(12)

# EUROPÄISCHE PATENTANMELDUNG

(21) Anmeldenummer: **79100171.2**

(22) Anmeldetag: **19.01.79**

(51) Int. Cl.²: **G 01 N 33/16**

---

(30) Priorität: **25.01.78 DE 2803109**

(43) Veröffentlichungstag der Anmeldung:
**08.08.79 Patentblatt 79/16**

(84) Benannte Vertragsstaaten:
**BE CH DE FR GB IT LU NL SE**

(71) Anmelder: **Zander, Rolf. Prof.Dr.**
**Reinhold-Schneider-Strasse 1**
**D-6500 Mainz(DE)**

(84) Benannte Vertragsstaaten:
**BE CH DE FR GB IT LU NL SE**

(71) Anmelder: **Lang, Werner, Dr.**
**Alter-Ruh-Weg**
**D-6500 Mainz(DE)**

(84) Benannte Vertragsstaaten:
**BE CH DE FR GB IT LU NL SE**

(71) Anmelder: **Wolf, Hans Uwe, Prof.Dr.**
**Lisztstrasse 10**
**D-7910 Neu-Ulm(DE)**

(84) Benannte Vertragsstaaten:
**BE CH DE FR GB IT LU NL SE**

(72) Erfinder: **Zander, Rolf. Prof.Dr.**
**Reinhold-Schneider-Strasse 1**
**D-6500 Mainz(DE)**

(72) Erfinder: **Lang, Werner, Dr.**
**Alter-Ruh-Weg**
**D-6500 Mainz(DE)**

(72) Erfinder: **Wolf, Hans Uwe, Prof.Dr.**
**Lisztstrasse 10**
**D-7910 Neu-Ulm(DE)**

(74) Vertreter: **Wehlmann, Gisela, Dipl.Chem.**
**C.H.-Boehringer Sohn Patentabteilung**
**D-6507 Ingelheim am Rhein(DE)**

---

(54) **Verfahren und Reagenz zur Bestimmung des Hämoglobingehaltes des Blutes.**

(57) Beansprucht wird ein Reagenz für die photometrische Bestimmung des Hämoglobingehaltes im Blut, das nach Zugabe der Blutprobe eine charakteristische Farbe zeigt. Dieses Reagenz besteht aus einer wässrig alkalischen Lösung, die ein wasserlösliches nicht-ionisches Detergens enthält. Diese Lösung bewirkt die Umwandlung aller Hämoglobin- und Häm-Derivate, die im Blut enthalten sind, in ein einziges Endprodukt, das ein bestimmtes Absorptionsmaximum im sichtbaren Spektralbereich aufweist.

Die Erfindung betrifft ferner das Verfahren zur Bestimmung des Hämoglobingehaltes im Blut unter Verwendung des oben erwähnten Reagenz.

EP 0 003 340 A2

Croydon Printing Company Ltd.

- 1 -

Die Erfindung betrifft ein Verfahren und ein Reagenz zur photometrischen Bestimmung des Hämoglobingehaltes des Blutes, das beim Eindosieren der Blutprobe eine charakteristische Verfärbung liefert.

Die quantitative Bestimmung des Hämoglobins im Blut ist eine der am häufigsten durchgeführten Analysen im Bereich der klinischen Chemie. Im Laufe dieses Jahrhunderts sind dazu verschiedene Verfahren entwickelt worden, die sich jedoch bezüglich Genauigkeit, Zuverlässigkeit und Reproduzierbarkeit erheblich voneinander unterscheiden (vgl. Lehrbücher der klinischen Chemie: HALLMANN, 1966; HENRY, 1964; RICHTERICH, 1971; HENRY et al., 1974; RICK, 1976):

1. Spektrophotometrische Verfahren (als Carboxy-Hämoglobin, Oxy-Hämoglobin oder Cyan-Hämiglobin, als Cyan-Hämatin, saures oder alkalisches Hämatin sowie als Pyridin-Hämochromogen);

2. Gasometrische Verfahren (Bestimmung der Sauerstoff- oder Kohlenmonoxid-Kapazität);

3. Chemische Verfahren (Bestimmung des Eisengehaltes);

4. Andere Verfahren (Refraktometrie, Dichtemessung, enzymatische Reaktionen wie die Pseudoperoxidaseaktivität des Hämoglobins).

Von diesen hat sich das spektrophotometrische Cyan-Hämiglobin-Verfahren durchgesetzt und eine weltweite Verbreitung gefunden. Nachdem in den Jahren 1953 bis 1963 praktisch alle Laboratorien dieses Verfahren anstelle anderer Verfahren eingeführt hatten (vgl. SPAANDER, 1964; SUNDERMAN, 1965) und ein Cyan-Hämiglobin-Standard empfohlen worden war (CANNAN, 1958), wurde dieses Verfahren von der Deutschen Gesellschaft für innere Medizin 1962 und vom International Committee for Standardization in Hematology (ICSH) 1967 empfohlen. Seit dieser Zeit gilt dieses Verfahren als Routineverfahren.

- 2 -

In Deutschland hat es die Funktion eines Referenzverfahrens, d.h. andere Verfahren dürfen nur angewandt werden, wenn das Verhältnis der damit erzielten Ergebnisse zum Ergebnis der Referenzmethode bekannt ist (DIN Vornorm 58 931).

Die Einführung des Cyan-Hämiglobin-Verfahrens als Standardverfahren hat dazu geführt, daß sich in der Fachwelt Vorurteile gegenüber anderen Methoden gebildet haben. "Hämiglobin-Cyanid ist das einzige bekannte stabile Hämoglobin-Derivat" (RICHTERICH, 1971); "alle übrigen Verfahren .. sind abzulehnen" (RICK, 1976); ".. Cyan-Hamiglobin (exaktester Nachweis)" (HALLMANN, 1966).

Das Prinzip des Cyan-Hämiglobin-Verfahrens besteht darin, daß das Hämoglobin mit Kalium-Ferricyanid zu Hämiglobin oxidiert und mit Kalium-Cyanid in Cyan-Hämiglobin übergeführt wird. Dabei wird als Reaktionslösung eine modifizierte sogenannte Drabkin'sche Lösung verwandt, wie sie von van KAMPEN und ZIJLSTRA ursprünglich angegeben wurde (van KAMPEN und ZIJLSTRA, 1961; ZIJLSTRA und van KAMPEN, 1960).

Die Vorteile dieses Verfahrens sind die folgenden:

1. Verwendung einer einzigen Reaktionslösung;

2. Alle Hämoglobin-Derivate des Blutes werden erfaßt (Desoxy-Hämoglobin, Oxy-Hämoglobin, Carboxy-Hämoglobin, Hämiglobin und weitgehend auch Sulf-Hämoglobin);

3. Das Cyan-Hämiglobin besitzt ein breites und flaches Extinktionsmaximum bei 540 nm, so daß auch mit anspruchslosen Filterphotometern zuverlässige Resultate erhalten werden können;

4. Das Lambert-Beer'sche Gesetz ist über einen weiten Meßbereich gültig;

5. Die Herstellung und der Versand von stabilen Standard-

- 3 -

Lösungen zu Kontrollzwecken ist möglich, sie können sowohl aus kristallinem Hämoglobin als auch aus gewaschenen Erythrocyten hergestellt werden;

6. Cyan-Hämiglobin ist ein stabiles Hämoglobin-Derivat, so daß die Messung der Extinktion nach einigen Minuten oder erst nach Tagen erfolgen kann.

Die Nachteile des Cyan-Hämiglobin-Verfahrens sind die folgenden (vgl. HENRY, 1964, S. 740):

1. Die Toxizität der Reaktionslösung verlangt besondere Maßnahmen bei deren Handhabung (Verwendung von Automaten-Pipetten, sorgfältige Beseitigung der Lösung im Ausguß);

2. Die Reaktionslösung ist nicht langzeitstabil, sie ist lichtempfindlich und muß entsprechend aufbewahrt werden (braune Flaschen o. ä.);

3. Die Konzentrationen der Reaktionspartner müssen genau eingehalten werden, dies gilt insbesondere für den Puffer, der einen definierten pH-Wert garantieren muß;

4. Die Reaktionszeiten der verschiedenen Hämoglobin-Derivate sind unterschiedlich und zum Teil erheblich zu lang. Ursprünglich wurde für alle Hämoglobin-Derivate eine Reaktionszeit von 3 Minuten angegeben (van KAMPEN und ZIJLSTRA, 1961), eine Aussage, die in den folgenden Jahren modifiziert wurde.

1965 weisen TAYLOR und MILLER nach, daß die Cyan-Hämiglobin-Bildung beim Vorliegen von Carboxy-Hämoglobin deutlich verlängert ist und dementsprechend erhebliche Fehler bei der Hämoglobin-Bestimmung auftreten müssen.
Van KAMPEN und ZIJLSTRA schlagen 1965 eine Reaktionszeit von 90 Minuten vor, falls mit Carboxy-Hämoglobin im zu untersuchenden Blut zu rechnen ist.
RODKEY weist 1967 nach, daß die Konvertierung von HbCO in

- 4 -

Cyan-Hämiglobin bei Zimmertemperatur 90 - 120 Minuten benötigt, während $HbO_2$ nur 10 Minuten beansprucht.

Dieser Autor kann die Reaktionszeit für die Umwandlung von HbCO zu Cyan-Hämiglobin auf 15 Minuten verkürzen, wenn die Konzentration von Kalium-Ferricyanid verfünffacht wird.

RICE (1967) löst dieses Problem dadurch, daß die Lösung auf $56^oC$ erhitzt wird. Die Reaktion von HbCO zu Cyan-Hämiglobin ist dann nach 3 - 5 Minuten abgeschlossen.

Daß Carboxy-Hämoglobin sehr viel langsamer zu Hämiglobin oxidiert wird als andere Derivate (Hb, $HbO_2$), wird zudem bei der sogenannten HOPPE-SEYLER'schen Probe für einen qualitativen HbCO-Nachweis im Blut benutzt (vgl. MASSMANN, 1954).

Weitere Fehlermöglichkeiten konnten ausgeschlossen werden. Zum Beispiel wurde eine Ausfällung von Plasma-Eiweißen und eine damit verbundene Trübung (vgl. z.B. GREEN und TEAL, 1959) dadurch vermieden, daß der Reaktionslösung ein Detergens zugegeben wurde (van KAMPEN und ZIJLSTRA, 1961).

Der Erfindung liegt die Aufgabe zugrunde, einen zuverlässigen quantitativen Hämoglobin-Nachweis zu entwickeln, der die genannten Vorteile des derzeitigen Cyan-Hämiglobin-Verfahrens besitzt und die genannten Nachteile desselben beseitigt.

Diese Aufgabe wird erfindungsgemäß mit einem Reagenz gelöst, das aus einer wässrigen, alkalischen Lösung besteht, der ein wasserlösliches, nicht-ionisches Detergens zugesetzt ist, wodurch alle (im Blut vorkommenden) Hämoglobin-Derivate und Häm-Derivate in ein einheitliches Endprodukt mit einem ausgeprägten Absorptionsmaximum im sichtbaren Spektralbereich überführt werden. Insbesondere werden solche Detergentien zugesetzt, die zu einem Endprodukt mit einem Absorptionsmaximum bei etwa 575 nm führen. Weiterbildungen und bevorzugte Ausführungsformen der Erfindung sind in den Unteransprüchen beschrieben.

Die Bestimmung des Hämoglobins als alkalisches Hämatin wurde erstmals 1922 von WU beschrieben. Allerdings zeichnete sich dieses Verfahren durch eine gewisse Umständlichkeit aus:

0003340

- 5 -

Hämoglobin wurde durch Zugabe von konzentrierter Salzsäure
in saures Hämatin umgewandelt, das anschließend durch Zugabe
von Natronlauge alkalisiert wurde. Die benötigte Zeit für
eine Bestimmung betrug deshalb auch etwa eine Stunde. Den
Vorteil gegenüber anderen Verfahren, wie saures Hämatin,
Methämoglobin oder Cyan-Hämiglobin, sah WU darin, daß Störungen durch Plasmabestandteile nicht auftraten.
CLEGG und KING (1942) gaben ein Verfahren zur Hämoglobin-
Bestimmung über das alkalische Hämatin an, bei welchem als
Reaktionslösung eine 0,1 n Natronlauge benutzt wurde, die
nach Zugabe des Blutes für 4 - 5 Minuten im Wasserbad von
100°C erhitzt wurde. Dieses Vorgehen erschien den Autoren
deshalb notwendig, da fetales Hämoglobin und alkaliresistentes Erwachsenen-Hämoglobin eine zu langsame Farbentwicklung zeigten.

Nach 1942 gab es eine Reihe von Autoren, die über zufriedenstellende Ergebnisse mit dem Verfahren alkalisches Hämatin
berichteten (KING et al., 1948; MacFARLANE et al., 1948;
DONALDSON et al., 1951; KING et al., 1951; SCAIFE, 1955).
Es wurden auch einige Modifizierungen vorgeschlagen, z.B.
die Verwendung einer anderen Wellenlänge (SCAIFE, 1955) oder
eines speziellen Standards (KING, 1947).
Andere Autoren hingegen verwarfen das Verfahren alkalisches
Hämatin wegen zu langer Reaktionszeiten oder störender Einflüsse durch Plasmabestandteile (PONDER, 1942; SUNDERMAN et al.,
1953). Die zur gleichen Zeit weit verbreitete Methode des
Cyan-Hämiglobins verdrängte das Verfahren alkalisches Hämatin
mehr und mehr, so daß später sogar auf eine "Besprechung des
alkalischen Hämatins verzichtet werden konnte" (LEGOWSKY und
BOROVICZENY, 1962).

Gegenüber dem klassischen Verfahren auf der Basis von alkalischem Hämatin unterscheidet sich das erfindungsgemäße Verfahren darin, daß ein alkalisches Hämatin mit besonderen Eigenschaften bezüglich
- Spektrum,
- Bildungsgeschwindigkeit und
- Haltbarkeit

- 6 -

entsteht, das im folgenden kurz als "alkalisches Hämatin
D - 575" bezeichnet wird.

Mit der Erfindung sind folgende Besonderheiten und Vorteile
verbunden:

1. Alle Häm- und Hämoglobin-Derivate werden quantitativ in
ein einheitliches Reaktionsprodukt, alkalisches Hämatin
D - 575, überführt und bei der photometrischen Messung
erfaßt: Hämoglobin, Oxy-Hämoglobin, Carboxy-Hämoglobin,
Hämiglobin, Sulf-Hämiglobin, Cyan-Hämiglobin, fetales
Hämoglobin sowie Chlorhämin.

2. Die Bildung des alkalischen Hämatins D - 575 nach Zugabe
der Probe zur Reaktionslösung ist nach spätestens 2 Minuten abgeschlossen und das Reaktionsprodukt zeigt über
Wochen die gleiche Extinktion. Dies gilt ohne Ausnahme
für alle unter 1. genannten Häm- und Hämoglobin-Derivate.

3. Die verwendete Reaktionslösung zeigt ideale Eigenschaften:
   a) Es wird nur eine Reaktionslösung benötigt.
   b) Die Reaktionslösung ist unbegrenzt haltbar.
   c) Die Handhabung der Reaktionslösung ist nahezu ungefährlich.
   d) Die Reaktionslösung kann bei Zimmertemperatur in üblichen Glas- oder Kunststoffgefäßen aufbewahrt werden.
   e) Die Reaktionslösung ist lichtunempfindlich.
   f) Die Reaktionslösung ist einfach herstellbar und billig.
   g) Die für die Reaktionslösung verwendeten Chemikalien sind
      austauschbar, d.h. es können verschiedene Chemikalien
      mit gleichem Erfolg eingesetzt werden.

   Es kann NaOH oder auch KOH verwendet werden; als
   Detergens können zum Beispiel verwendet werden:
   Polyäthylenglycol-p-t-octylphenoläther (insbesondere
   Polyäthylenglycol-mono-[p,(1,1,3,3-tetramethylbutyl)-
   phenyl]-äther, wie es unter dem Handelsnamen Triton
   X-100 erhältlich ist),

- 7 -

Polyäthylenglycollauryläther, Polyäthylenglycolcetyläther, Polyäthylenglycololeyläther und Polyäthylenglycolstearyläther.

h) Die Konzentration der Reaktionspartner NaOH und Detergens ist unkritisch, d.h. eine erhebliche Abweichung
von den optimalen Bedingungen hat praktisch keinen Einfluß auf die Durchführbarkeit des Verfahrens. Wird die
NaOH im Konzentrationsbereich von 0,05 bis 0,5 m und
die des Detergens von 1 bis 4 % geändert, so ändert
sich das spektrale Verhalten des Reaktionsproduktes
nicht, lediglich die für die Bestimmung notwendige Reaktionszeit wird von 1 - 2 Minuten auf 5 - 10 Minuten
verlängert.

i) Der pH-Wert (s.o. NaOH-Konzentration) und die Temperatur haben in einem weiten Bereich keinen Einfluß auf die
Durchführbarkeit des Verfahrens.

k) Im Gegensatz zu verdünntem Blut ist das Reaktionsprodukt
alkalisches Hämatin D - 575 intensiv grün gefärbt (Farbeindruck bei einer Lichtweglänge von 1 - 2 cm). Daraus
resultiert, daß der Abschluß der Reaktion zumindest grob
mit dem Auge beurteilt werden kann.

l) Das alkalische Milieu der Reaktionslösung eignet sich
besonders gut für einen Hämoglobin-Nachweis (vgl. BARER
und GAFFNEY, 1957): Die Erythrocyten werden vollständig
hämolysiert, die Erythrocytenmembranen werden aufgelöst
und geben das an sie adsorbierte Hämoglobin frei,
schließlich wird eine Ausfällung der Plasmaproteine
(Trübung) verhindert.

4. Das Reaktionsprodukt alkalisches Hämatin D - 575 zeigt
indeale Eigenschaften:
a) Die Extinktion ist groß genug, um eine Hämoglobin-
Bestimmung mit nur 10 - 20 Mikroliter Blut (bei Verwendung üblicher Mengen von Reaktionslösung, nämlich
2 - 5 ml) vornehmen zu können.

- 8 -

b) Das Lambert-Beer'sche Gesetz ist über einen weiten Bereich gültig.

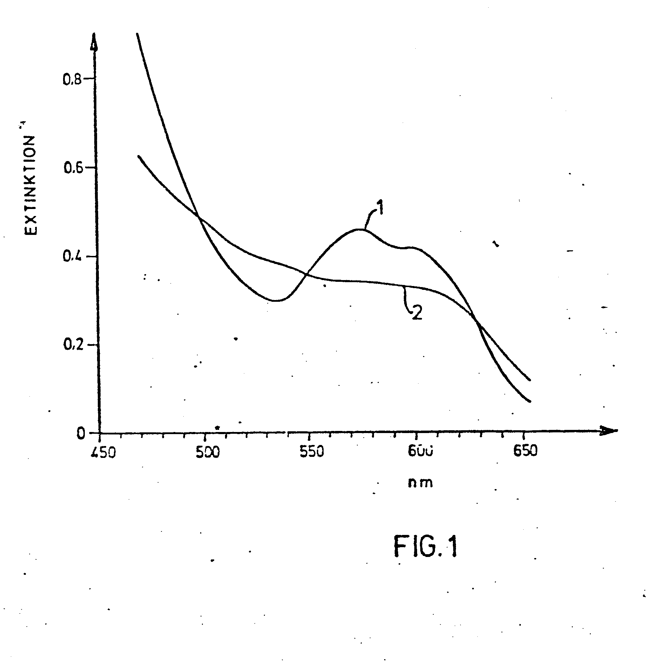

c) Das Spektrum des Reaktionsproduktes zeigt ein breites Maximum bei einer Wellenlänge von 575 nm (daher die Bezeichnung alkalisches Hämatin D - 575), so daß auch mit anspruchslosen Filterphotometern zuverlässige Bestimmungen durchgeführt werden können. Das Spektrum ist unabhängig von den Konzentrationen der Reaktionspartner im o.g. Bereich, es unterscheidet sich deutlich von dem des alkalischen Hämatins ohne Zusatz eines Detergens. Das spektrale Verhalten des alkalischen Hämatins D - 575 ist im Vergleich zum Spektrum des alkalischen Hämatins in Abb. 1 wiedergegeben. Gewonnen wurden beide Spektren, indem eine Cyan-Hämiglobin-Standardlösung (212,4 mg/100 ml) mit jeweils dem gleichen Volumen 0,1 m NaOH (Bildung von alkalischem Hämatin) beziehungsweise 0,1 m NaOH mit einem Zusatz von 2,5 % Decaäthylenglycol-p-t-octylphenyläther (Bildung von alkalischem Hämatin D - 575) verdünnt wurde. Bei dieser Zusammensetzung erhält man optimale Ergebnisse hinsichtlich der Reaktionszeit. Während das alkalische Hämatin in NaOH das in der Literatur mehrfach beschriebene Spektrum mit einem Plateau zwischen 550 und 600 nm zeigt (vgl. z.B. HUNTER, 1951), weist das alkalische Hämatin D - 575 im Beisein eines nicht-ionischen Detergens ein breites Maximum bei 575 nm auf.

Dieser Effekt wird durch ionische Detergentien nicht erbracht:
Während anionische Detergentien, wie z.B. das Natrium-Dodecylsulfat, die charakteristische Bande bei 575 nm nicht erzeugt, tritt bei Verwendung kationischer Detergentien, wie Benzyl-diisobutyl-phenoxy-äthoxy-dimethylammoniumchlorid oder Dodecyl-dioxyäthyl-benzyl-ammoniumchlorid bei Zugabe von Blut ein brauner Niederschlag auf.
Es muß sich bei der Wirkung des nicht ionischen Detergens um einen unspezifischen Einfluß handeln, da er von der Struktur desselben unabhängig ist (bezüglich der Änderung des Hämoglobin-Spektrums durch indifferente Lösungsmittel, vgl. HEILMEYER, 1933, S. 116).

5. Da das alkalische Hämatin D - 575 sehr stabil ist, können Standardlösungen mit bekannter Konzentration aus gewaschenen Erythrocyten, aus kristallinem Hämoglobin und aus kristallinem Chlor-Hämin hergestellt werden.
Insbesondere das letzte Verfahren ist für die Herstellung eines Standards sehr zu empfehlen, da Chlor-Hämin eine einfache, genau definierte Substanz ist (MG 651,97, Summenformel $C_{34}H_{32}Cl\ Fe\ N_4O_4$), in der beschriebenen Reaktionslösung sehr gut löslich ist und eindeutig die typischen spektralen Eigenschaften des alkalischen Hämatins D - 575 aufweist, d.h. es wird quantitativ in alkalisches Hämatin D 575 überführt.
Als besonderer Vorteil kann gelten, daß die weltweit verbreiteten Cyan-Hämiglobin-Standardlösungen ebenfalls nach Verdünnung mit der Reaktionslösung benutzt werden können (Fig. 1). Das bedeutet, daß eine Standardisierung des neuen Verfahrens mit bereits existierenden Standards vorgenommen werden kann.

Im folgenden soll ein Ausführungsbeispiel beschrieben werden.

Ein Volumen von 20 $\mu$l Blut wird mit einer kalibrierten Kapillare oder einer sogenannten Sahli-Pipette vollständig in eine Einweg-Meßküvette überführt, die 3 ml Reaktionsflüssigkeit enthält. Die Meßküvette besteht z.B. aus glasklarem Polystyrol und besitzt eine Kantenlänge von 1 cm.
An ihrem oberen Ende befindet sich eine Kunststoffabschlußkappe, die normalerweise verschlossen ist und erst kurz vor dem Meßvorgang geöffnet wird. Die Reaktionslösung in der Meßküvette bleibt über Monate stabil, so daß die Küvetten dementsprechend lange gelagert werden können.
Die Reaktionslösung besteht z.B. aus 0,1 m NaOH in Wasser mit einem Zusatz von 2,5 % Decaäthylenglycol-p-t-octylphenyläther. Dieses Detergens hat sich besonders bewährt, da es in flüssiger Form vorliegt und daher einfach zu handhaben ist. Nach dem Einbringen der Blutprobe wird das Blut durch Schütteln der Meßküvette mit der Reaktionslösung vermischt. Dabei setzt die Umwandlung aller Hämoglobin-Derivate schlagartig ein. Die Lösung färbt sich sofort intensiv grün. Die Messung der Extinktion im Photometer bei einer Wellenlänge von 575 nm kann nach 1, spätestens nach 2 Minuten, erfolgen. Da die Extinktion über Tage stabil bleibt, kann die Messung auch später vorgenommen werden. Als Photometer können handelsübliche Filterphotometer benutzt werden, wobei das Filter so gewählt wird, daß ein schmaler Bereich bei 575 nm durchgelassen wird. Ein geeignetes Filterphotometer ist z.B. in der deutschen Auslegeschrift 2 448 206 beschrieben.

Wird Blut mit einer normalen Hämoglobin-Konzentration von etwa 16 g/100 ml verwendet, so ergibt sich bei diesem Verdünnungsverhältnis eine Extinktion von ca. 0,46 bei der genannten Wellenlänge, gemessen gegen eine mit Wasser gefüllte Küvette der gleichen Schichtlänge (1 cm).

Der Vergleich mit einer Standardlösung (z.B. einer 0,1 mM Chlor-Hämin-Lösung in der gleichen Reaktionslösung oder einer Cyan-Hämiglobinlösung bekannter Konzentration, verdünnt mit dem gleichen Volumen der Reaktionslösung) ergibt die gewünschte Hämoglobin-Konzentration in g/100 ml.

0003340

- 11 -

Literatur

BARER, R. und GAFFNEY, F. M.:
　　　　The use of dilute alkaline in haemoglobinometry
　　　　Clin. Chim. Acta 2, 140 - 156 (1957)

Beschluß der Deutschen Gesellschaft für Innere Medizin betreffend Standardisierung der Hämoglobin-Bestimmung
　　　　Ärztl. Labor 8, 188 (1962)

CANNAN, R. K.:
　　　　Proposal for a certified standard for use in
　　　　hemoglobinometry　- second and final report
　　　　J. Lab. Clin. Med. 52, 471 - 476 (1958)

CLEGG, J.W. und KING, E. J.:
　　　　Estimation of haemoglobin by the alkaline
　　　　haematin method
　　　　Brit. Med. J. 2, 329 - 333 (1942)

Deutsche Normen:
　　　　Bestimmung des Hämoglobingehaltes im Blut.
　　　　DIN Vornorm 58 931 (1970)

DONALDSON, R., SISSON, R.B., KING, E.J., WOOTTON, I.D.P.,
MacFARLANE, R.G.: Determination of haemoglobin.
　　　　VII. Standardized optical data for absolute
　　　　estimation.
　　　　Lancet 1, 874 - 881 (1951)

MacFARLANE, R.G., KING, E.J., WOOTTON, I.D.P., GILCHRIST, M.:
　　　　Determination of haemoglobin.
　　　　III. Reliability of clinical and other methods.
　　　　Lancet 2, 282 - 286 (1948)

- 12 -

GREEN, P. und TEAL, C.F.J.:
Modification of the cyanmethemoglobin reagent
for analysis of hemoglobin in order to avoid
precipiation of globulins.
Am. J. Clin. Pathol. 32, 216 - 217 (1959)

HALLMANN, L.: Klinische Chemie und Mikroskopie (10. Aufl.)
Thieme, Stuttgart 1966
HEILMEYER, L.: Medizinische Spektrophotometrie
Fischer, Jena 1933
HENRY, R. J.: Clinical Chemistry (1$^{st}$ ed.)
Harper & Row, New York 1964
HENRY, R.J., CANNON, D.C., WINKELMAN, J.W.:
Clinical Chemistry (2$^{nd}$ ed.)
Harper & Row, New York 1974
HUNTER, F. T.:
The quantitation of mixtures of hemoglobin
derivatives by photoelectric spectrophotometry.
Thomas, Springfield 1951
International Committee for Standardization in Haematology.
Recommendations for haemoglobinometry in
human blood.
Brit. J. Haemat. 13, 71 - 75 (1967)
van KAMPEN, E.J. und ZIJLSTRA, W.G.:
Standardization of hemoglobinometry.
II. The hemoglobincyanide method.
Clin. Chim. Acta 6, 538 - 544 (1961)
van KAMPEN, E.J. und ZIJLSTRA, W.G.:
In: Advances in Clinical Chemistry, Vol. 8
p. 160 (Sobotka, H., Stewart, C.P., eds.)
Academic Press, New York 1965
KING, E.: Alkaline haematin method for haemoglobin
determination.
Brit. Med. J. 2, 349 - 350 (1947)

- 13 -

KING, E.J., GILCHRIST, M., WOOTTON, I.D.P., DONALDSON, R., SISSON, R.B., MacFARLANE, R.G., JOPE, J.M., O'BRIEN, J.R.P., PETERSON, J.M., STRANGEWAYS, D.H.:
Determination of haemoglobin.
V. Precision of colorimetric methods.
Lancet 2, 563 - 566 (1948)

KING, E.J., BARTHOLOMEW, R.J., GEISER, M., VENTURA, S., WOOTTON, I.D.P., MacFARLANE, R.G., DONALDSON, R., SISSON, R.B.:
Determination of haemoglobin.
VIII. Accuracy of methods applied to abnormal bloods.
Lancet 1, 1044 - 1045 (1951)

LEGOWSKI, S. und v. BOROVICZENY, K.G.:
Exakte Hämoglobinbestimmung in der täglichen Praxis.
Dtsch. Med. Wschr. 87, 1953 - 1960 (1962)

MASSMANN, W. : Einige qualitative Kohlenoxydbestimmungen im Blut. - Dtsch.Med.Wschr. 79, 1140-1142 (1954)

PONDER, E.: Errors effecting the acid and the alkali hematin methods of determination hemoglobin.
J. biol. Chem. 144, 339 - 342 (1942)

RICE, E.W.: Rapid determination of total hemoglobin as hemoglobin cyanide in blood containing carboxyhemoglobin.
Clin. Chim. Acta 18, 89 - 91 (1967)

RICHTERICH, R.: Klinische Chemie (3. Aufl.)
Karger, Basel 1971

RICK, W.: Klinische Chemie und Mikroskopie (4. Aufl.)
Springer, Berlin/Heidelberg/New York 1976

RODKEY, F.L.: Kinetic aspects of cyanmethemoglobin formation from carboxyhemoglobin.
Clin. Chem. 13, 2 - 5 (1967)

SCAIFE, J.F.: Stability of alkaline solutions of haematin.
Analyst 80, 562 - 565 (1955)

SPAANDER, J.: Die Verwertung von Blutuntersuchungen Strahlenschutz i. Forschung und Praxis 4, 122 - 127 (1964)

SUNDERMAN, F.W.:

Status of clinical hemoglobinometry in the
United States
Am. J. Clin. Pathol. 43, 9 - 15 (1965)

SUNDERMAN, F.W., MacFATE, R.P., MacFADYEN, D.A., STEVENSON,G.F.,
COPELAND, G.E.:

Symposium on clinical hemoglobinometry
Am. J. Clin. Pathol. 23, 519 - 598 (1953)

TAYLOR, J.D. und MILLER, J.D.M.:

A source of error in the cyanmethemoglobin method
of determination of hemoglobin concentration in
blood containing carbon monoxide.
Am. J. Clin. Pathol. 43, 265 - 271 (1965)

WU, H.: Studies on hemoglobin.

I. The advantage of alkaline solutions for
colorimetric determination of hemoglobin
J. Biochem. 2, 173 - 180 (1922)

ZIJLSTRA, W.G. und van KAMPEN, E.J.:

Standardization of hemoglobinometry.
I. The extinction coefficient of hemoglobincyanide
at $\lambda$ = 540 m/u : $\varepsilon_{HiCN}^{540}$

Clin. Chim. Acta 5, 719 - 726 (1960)

- 1 -

P a t e n t a n s p r ü c h e

1. Reagenz zur photometrischen Bestimmung des Hämoglobingehaltes des Blutes, das beim Eindosieren der Blutprobe eine charakteristische Verfärbung liefert, gekennzeichnet durch eine wässrige, alkalische Lösung, der ein wasserlösliches, nicht-ionisches Detergens zugesetzt ist, wodurch alle (im Blut vorkommenden) Hämoglobin-Derivate und Häm-Derivate in ein einheitliches Endprodukt mit einem ausgeprägten Absorptionsmaximum im sichtbaren Spektralbereich überführt werden.

2. Reagenz nach Anspruch 1, dadurch gekennzeichnet, daß das Absorptionsmaximum bei etwa 575 nm liegt.

3. Reagenz nach Anspruch 1 bis 2, dadurch gekennzeichnet, daß die alkalische Reaktionslösung eine 0,05 - 0,5 molare Natron- oder Kalilauge ist, der 0,5 - 10 Gew.% Detergens zugesetzt sind.

4. Reagenz nach Anspruch 1 bis 3, dadurch gekennzeichnet, daß das Detergens ein Polyäthylenglycol-p-alkylphenyläther der allgemeinen Formel $H-(O-CH_2-CH_2)_x-O-\langle\_\rangle-R$ ist, wobei $7 \leq x \leq 12$ und R einen Alkylrest vom Typ Methyl, Äthyl, Propyl, Butyl, Pentyl, Hexyl, Heptyl, Octyl, Nonyl, Decyl, Undecyl und Dodecyl bedeutet, oder ein Polyäthylenglycollauryl-, -cetyl-, -oleyl- oder -stearyläther ist.

5. Reagenz nach Anspruch 4, dadurch gekennzeichnet, daß das Detergens aus Decaäthylenglycol-p-t-octylphenyläther besteht.

6. Verfahren zur Bestimmung des Hämoglobingehaltes einer Blutprobe, unter Verwendung eines Reagenz gemäß den Ansprüchen 1 bis 5, bei dem die Probe durch Hinzufügen einer alkalischen, wässrigen Lösung hämolysiert wird und das alkalische Hämatin photometrisch bestimmt wird, dadurch gekennzeichnet, daß der wässrigen alkalischen Lösung ein

- 2 -

wasserlösliches, nicht-ionisches Detergens zugesetzt wird, wodurch alle (im Blut vorkommenden) Hämoglobin-Derivate und Häm-Derivate in ein einheitliches Endprodukt mit einem ausgeprägten Absorptionsmaximum im sichtbaren Spektralbereich überführt werden, bei dem die photometrische Messung durchgeführt wird.

7. Verfahren nach Anspruch 6, dadurch gekennzeichnet, daß durch die Reaktion ein alkalisches Hämatin gebildet wird, das ein Absorptionsmaximum bei einer Wellenlänge von etwa 575 nm aufweist.

8. Verfahren nach Anspruch 6 bis 7, dadurch gekennzeichnet, daß als alkalische Lösung eine 0,05 bis 0,5 molare Natron- oder Kalilauge verwendet wird, der 0,5 bis 10 Gew% Detergens zugesetzt sind.

9. Verfahren nach Anspruch 6 bis 8, dadurch gekennzeichnet, daß als Detergens ein Polyäthylenglycol-p-alkylphenyläther, Polyäthylenglycollauryläther, Polyäthylenglycolcetyläther, Polyäthylenglycololeyläther oder Polyäthylenglycolstearyläther verwendet wird.

FIG.1